# EUROPEAN PATENT APPLICATION

(11) **EP 4 494 587 A1**
(43) Date of publication of application: **22.01.2025**
(21) Application number: 22931786.2
(22) Date of filing: 11.11.2022
(51) Int. Cl.: A61B 18/14

(54) **TEMPERATURE CONTROL METHOD, APPARATUS, AND DEVICE FOR PULMONARY ARTERY RADIO FREQUENCY ABLATION SYSTEM**

(30) Priority: 18.03.2022 CN 202210272558
(71) Applicant: Pulnovo Medical (Wuxi) Co., Ltd., Xishan Economic Development Zone Wuxi Jiangsu 214191 (CN)
(72) Inventor: ZHAO, Sukai, Wuxi, Jiangsu 214191 (CN); WEI, Jiangshan, Wuxi, Jiangsu 214191 (CN); ZHOU, Yang, Wuxi, Jiangsu 214191 (CN)
(74) Representative: Mathys & Squire
(86) International application number: PCT/CN2022/131541
(87) International publication number: WO 2023/173774

(57) **Abstract**

The present disclosure is directed to medical devices and provides a temperature control method, device, electronic device, and computer-readable storage medium for a pulmonary artery radio frequency ablation system. The radio frequency ablation system can comprise a radio frequency power source, an ablation electrode and a temperature sensing wire coupled with the ablation electrode. The method can comprise, during each of a plurality of ablation cycles, obtaining a current temperature measurement value measured by the temperature sensing wire and comparing the current temperature measurement value with a preset target temperature. In some instances, the method can comprise controlling the radio frequency power source to supply a power to the ablation electrode when the current temperature measurement value is lower than the preset target temperature during the ablation cycle, comparing the current temperature measurement value with a previous temperature measurement value during the ablation cycle when the current temperature measurement value is higher than or equal to the preset target temperature, and controlling the radio frequency power source to suspend a power supply to the ablation electrode when the current temperature measurement value is higher than the previous temperature measurement value.

## Description

### CROSS-REFERENCES TO RELATED APPLICATIONS

The present application claims priority from Chinese Patent Application No. 202210272558.X filed on March 18, 2022, the content of which is incorporated herein by reference in its entirety.

### TECHNICAL FIELD

The present disclosure relates to the technical field of medical devices, in particular to a temperature control method, apparatus, electronic device, computer-readable storage medium and computer program product for a pulmonary artery radio frequency ablation system.

### BACKGROUND

Pulmonary artery disease, such as pulmonary hypertension, is regarded as a challenging condition to treat. While the prevalence of primary pulmonary hypertension is relatively low, pulmonary hypertension secondary to pulmonary interstitial fibrosis, connective tissue disease, portal hypertension, chronic pulmonary embolism and left heart disorder is highly prevalent, with a five-year mortality reaching up to 30 %. Therefore, treatment of pulmonary hypertension is of importance.

Radio frequency ablation catheters can be used in the treatment of pulmonary artery diseases, such as pulmonary hypertension, by positioning the radio frequency ablation catheter at the pulmonary artery, thereby enabling the application of radio frequency heating to body tissues (e.g., sympathetic nerves) on the pulmonary artery. The temperature of the radio frequency heating is of importance with regard to the therapeutic effect of the radio frequency ablation.

The approaches described in this section are not necessarily approaches that have been previously contemplated or employed in the prior art. It should be noted that, unless otherwise indicated, no approach described in this section is considered to be prior art merely because it is described in this section. Similarly, unless otherwise indicated, the problems addressed in this section should not be assumed to have been previously identified in any prior art.

### SUMMARY

The present disclosure provides a temperature control method, device, electronic device, computer-readable storage medium and computer program product for a pulmonary artery radio frequency ablation system.

An aspect of the present disclosure provides a temperature control method for a pulmonary artery radio frequency ablation system. The radio frequency ablation system can comprise a radio frequency power source, an ablation electrode and a temperature sensing wire coupled with the ablation electrode. In some embodiments, the method can comprise, during each of a plurality of ablation cycles, obtaining a current temperature measurement value measured by the temperature sensing wire and comparing the current temperature measurement value with a preset target temperature. In some instances, the method can comprise controlling the radio frequency power source to supply a power to the ablation electrode when the current temperature measurement value is lower than the preset target temperature during the ablation cycle, comparing the current temperature measurement value with a previous temperature measurement value during the ablation cycle when the current temperature measurement value is higher than or equal to the preset target temperature, and controlling the radio frequency power source to suspend a power supply to the ablation electrode when the current temperature measurement value is higher than the previous temperature measurement value.

Another aspect of the present disclosure provides a temperature control device for a pulmonary artery radio frequency ablation system. The radio frequency ablation system can comprise a radio frequency power source, an ablation electrode and a temperature sensing wire coupled with the ablation electrode. In some embodiments, the temperature control device can comprise a temperature measurement value acquisition unit, a first comparison unit, a second comparison unit and a control unit. The temperature measurement value acquisition unit can be configured to obtain a current temperature measurement value measured by the temperature sensing wire during each of a plurality of ablation cycles. The first comparison unit can be configured to compare the current temperature measurement value with a preset target temperature during each of the plurality of ablation cycles. The control unit can be configured to control the radio frequency power source to supply a power to the ablation electrode during the ablation cycle when the current temperature measurement value is lower than the preset target temperature. The second comparison unit can be configured to compare the current temperature measurement value with a previous temperature measurement value during the ablation cycle when the current temperature measurement value is higher than or equal to the preset target temperature. The control unit can be further configured to control the radio frequency power source to suspend a power supply to the ablation electrode when the current temperature measurement value is greater than the previous temperature measurement value.

Another aspect of the present disclosure provides an electronic device. The electronic device can comprise at least one processor and a memory in communication with the at least one processor. The memory can store therein instructions executable by the at least one processor that, upon execution by the at least one processor, implements the temperature control method for pulmonary artery radio frequency ablation system.

Another aspect of the present disclosure provides a non-transitory computer-readable storage medium storing computer instructions. The computer instructions can be executable to cause a computer to implement the temperature control method for pulmonary artery radio frequency ablation system.

Another aspect of the present disclosure provides a computer program product comprising a computer program, upon execution by the at least one processor, implements the temperature control method for pulmonary artery radio frequency ablation system.

Embodiments of the present disclosure can reduce the fluctuations in the temperature of the radio frequency ablation electrode and stabilize the temperature of the radio frequency ablation electrode within a specified range.

It should be noted that the description provided in this section is not intended to identify the most significant or essential characteristics of the embodiments of the present disclosure. Furthermore, it is not intended to restrict the scope of the present disclosure. The following specification will aid in understanding the other features of the present disclosure.

### BRIEF DESCRIPTION OF THE DRAWINGS

The accompanying drawings illustrate exemplary embodiments and form part of the specification. The drawings are used in conjunction with the textual description of the specification to explain exemplary implementations of the embodiments. It should be noted that the illustrated embodiments are for illustrative purposes only and do not limit the scope of the claims. In all of the accompanying drawings, the same reference number refer to similar, but not necessarily identical, elements. In the drawings:
**FIG. 1** illustrates a schematic diagram of an exemplary system in which a temperature control method for a pulmonary artery radio frequency ablation system according to embodiments of the present disclosure can be implemented;
**FIG. 2** illustrates a flowchart of a temperature control method for a pulmonary artery radio frequency ablation system according to embodiments of the present disclosure;
**FIG. 3** illustrates a flowchart of a temperature control method for a pulmonary artery radio frequency ablation system according to embodiments of the present disclosure;
**FIG. 4** illustrates a flowchart of a temperature control method for a pulmonary artery radio frequency ablation system according to embodiments of the present disclosure;
**FIG. 5** illustrates a flowchart of a temperature control method for a pulmonary artery radio frequency ablation system according to embodiments of the present disclosure;
**FIG. 6** illustrates a block diagram of a temperature control device for a pulmonary artery radio frequency ablation system according to embodiments of the present disclosure; and
**FIG. 7** illustrates a block diagram of an exemplary electronic device capable of implementing embodiments of the present disclosure.

### DETAILED DESCRIPTION

Exemplary embodiments of the present disclosure are described below with reference to the accompanying drawings. The description contains numerous details of the embodiments, with the aim of facilitating understanding. It should be noted that the embodiments are provided for illustrative purposes only. It should be noted that various changes and modifications can be made to the embodiments without deviating from the scope of the present disclosure. Similarly, descriptions of well-known features and structures are omitted from the following description for the sake of clarity and brevity.

Unless otherwise indicated, the use of terms "first" "second" in the present disclosure to describe various elements is not intended to limit the positional, temporal, or importance relationships of the elements. Rather, these terms are employed solely for the purpose of distinguishing one element from another. In some instances, the first element and second element may be indicative of the same instance of the element. In other cases, however, based on the contextual description, they may also be indicative of different instances.

The terminology used in describing the various described examples in this disclosure is for the purpose of describing particular examples only and is not intended to be limiting. Unless the context clearly indicates otherwise, if the number of elements is not specifically limited, there may be one or more elements. In addition, the term "and/or" used in the present disclosure covers any one and all possible combinations of the listed items.

As discussed hereinabove, radio frequency ablation catheters can be used to treat pulmonary artery disease such as pulmonary arterial hypertension. This can be achieved by applying radio frequency heating to the target area by placing a radio frequency ablation electrode at the target site within the pulmonary artery. The temperature of the radio frequency heating can be important with regard to the therapeutic effect of the radio frequency ablation.

The target point temperature of the radio frequency ablation is challenging to regulate and often exceeds the safety threshold during percutaneous pulmonary artery denervation. As the patient is conscious of the chest cavity while under local anesthesia at the incision during the procedure, a fit between the ablation electrode and the target point of the pulmonary artery may vary depending on to the patient's condition. This can result in tissue denaturation or carbonization, as the target point of the tissue exceeds the safe temperature. Moreover, the impedance of the carbonized tissue is excessively high, which causes the carbonized tissue to coagulate on the surface of the ablation electrode and subsequently affecting the output of the radio frequency energy.

In prior art, the radio frequency ablation system can be controlled via a power control mode. In order to stabilize the output power of the radio frequency ablation system at a specified value, the operator can specify a desired output power in the power control mode and subject the radio frequency ablation system to a preset adjustment process for a certain interval. The target point temperature, however, serves merely as a safety threshold. In practice, prior to reaching the desired temperature, the actual ablation process may require several rounds of power value adjustments. In some cases, the temperature may fluctuate and fail to be stabilized at the desired value.

Also, in prior art, the radio frequency ablation system can be controlled via a temperature control mode. In order to stabilize the target point temperature of the radio frequency ablation system at a specified temperature or temperature range the temperature control mode, the operator can specify a desired temperature or temperature range and subject the radio frequency ablation system to a preset adjustment process for a certain interval. In practice, however, due to inherent limitations of the algorithm and circuit design, the target point temperature may exhibit fluctuation or be constrained to a relatively broad temperature range.

Inventors of the present disclosure realized that, due to the hysteresis observed in the temperature response of the human pulmonary artery tissue, the temperature control approaches in the prior art are incapable of effectively controlling the temperature of the radio frequency ablation electrode. Consequently, these approaches are incapable of maintaining precise control over the temperature at the target point of the pulmonary artery. In practice, the target point temperature of the radio frequency ablation may be regulated excessively, resulting in significant fluctuations in temperature. Additionally, the stabilization of the target point temperature of the radio frequency ablation may require an excessive amount of time, or the controller may even become destabilized. Such outcomes have an adverse effect on the results of radio frequency ablation.

In view of above technical challenges in the prior art, the present disclosure proposes a temperature control method for a pulmonary artery radio frequency ablation system in consideration of the hysteresis observed in the temperature response of human pulmonary artery tissues. The method of present disclosure can reduce the fluctuations in the temperature of the radio frequency ablation electrode and stabilize the temperature of the radio frequency ablation electrode within a specified range, thereby reducing the time required for the electrode to reach a steady state and preventing a destabilization in the control of the electrode temperature.

Embodiments of the present disclosure will be described in detail with reference to the drawings.

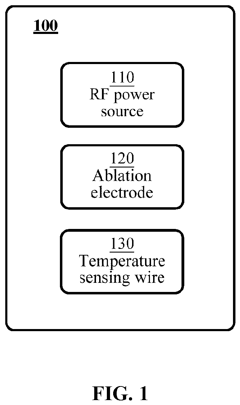

**FIG. 1** illustrates a schematic diagram of an exemplary system 100 in which the temperature control method for pulmonary artery radio frequency ablation system according to embodiments of the present disclosure can be implemented.

As shown in **FIG. 1****,** the radio frequency ablation system 100 can comprise a radio frequency power source 110, an ablation electrode 120, and a temperature sensing wire 130 coupled to the ablation electrode 120. The radio frequency power source 110 can be configured to provide a power (e.g., a radio frequency power) to the ablation electrode 120, enabling the ablation electrode 120 to perform a radio frequency ablation. The temperature sensing wire 130 can be configured to measure a temperature at the ablation electrode 120.

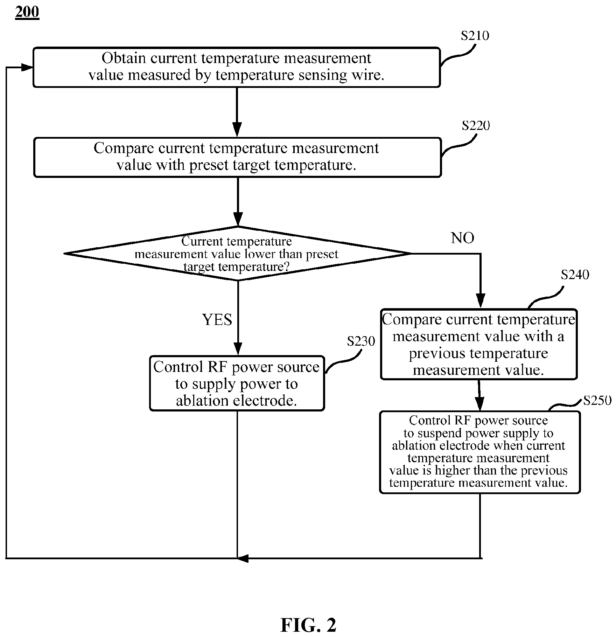

**FIG. 2** illustrates a flowchart of a temperature control method 200 for a pulmonary artery radio frequency ablation system according to embodiments of the present disclosure. As shown in **FIG. 2****,** the method 200 can comprise, for each of a plurality of ablation cycles, steps S210 to S250.

In step S210, a current temperature measurement value measured by the temperature sensing wire 130 can be obtained.

In step S220, the current temperature measurement value can be compared with a preset target temperature.

In step S230, the radio frequency power source 110 can be controlled to supply a power to the ablation electrode 120 when the current temperature measurement value is lower than the preset target temperature during the ablation cycle.

When the current temperature measurement value is higher than or equal to the preset target temperature, in step S240, the current temperature measurement value can be compared with a previous temperature measurement value during the ablation cycle. In step S250, the radio frequency power source 110 can be controlled to suspend a power supply to the ablation electrode 120 when the current temperature measurement value is higher than the previous temperature measurement value.

Due to the hysteresis in the temperature response of the human pulmonary artery tissue, even if the temperature of the ablation electrode 120 is lowered or the ablation heating of the pulmonary artery target is terminated, the temperature of the pulmonary artery target may continue to increase. This hysteresis may render the conventional temperature control approach (e.g., Proportional Integral Derivative (PID) control approach) slow to stabilize the temperature, or even incapable of adjusting the temperature, thereby leading to a destabilization in the temperature control.

Unlike the prior art temperature control approaches, the method 200 of the present disclosure can compare a temperature measurement value at the current moment with a temperature measurement value at a previous moment and. This comparison can allow for the radio frequency power source 110 to suspend the energy supply to the ablation electrode 120 when the temperature measurement value at the current moment is greater than the temperature measurement value at the previous moment. In consideration of the hysteresis in the temperature response of the human pulmonary artery tissue, it is feasible to control the radio frequency power source 110 to halt the supply of energy to the ablation electrode 120 in a prompt manner in the event that the temperature measurement value of the current moment exceeds that of the previous moment. Consequently, the method of the present disclosure is capable of rapidly adjusting the system to a stable state and preventing a destabilization in the temperature control. Furthermore, the method of the present disclosure can prevent an excessive heating of the target in the pulmonary artery.

A comparison revealed that the time required to regulate the system to a steady state was reduced by more than 50% when the temperature control of a pulmonary artery radio frequency ablation system was performed by the method of the present disclosure, as opposed to the conventional PID control approach.

In some embodiments, the preset target temperature in step S220 can be a target temperature determined based on the medical properties of the pulmonary artery tissue. In some instances, the preset target temperature can be 43 degrees Celsius.

In some embodiments, following one cycle of ablation, the method 200 can be performed in the subsequent cycle of ablation from step S210. This enables the performance of a plurality of ablation cycles until the radio frequency ablation procedure is completed.

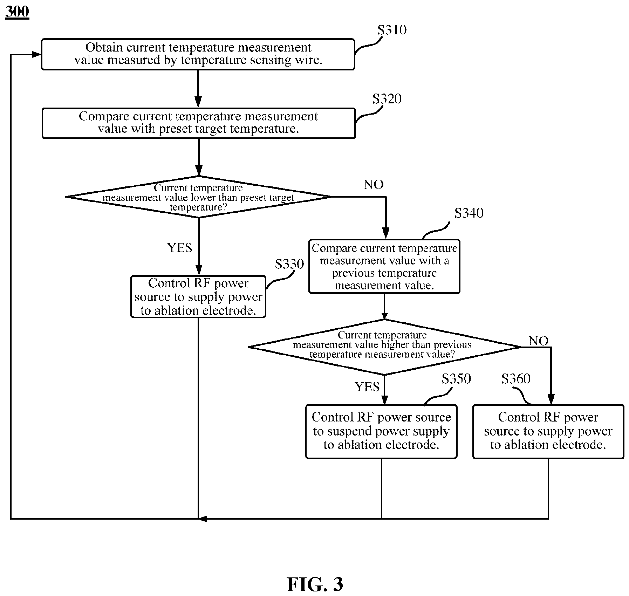

**FIG. 3** illustrates a flowchart of a temperature control method 300 for a pulmonary artery radio frequency ablation system according to embodiments of the present disclosure. As shown in **FIG. 3****,** the method 300 can comprise steps S310 to steps S360. The steps S310 to steps S350 in the method 300 can be similar to steps S210 to steps S250 in the method 200 as described hereinabove with respect to **FIG. 2****.** Therefore, description on the steps S310 to steps S350 will be omitted for brevity.

In some embodiments, the method 200 can further comprise, in step S360, controlling the radio frequency power source 110 to supply a power to the ablation electrode 120 when the current temperature measurement value is lower than or equal to the previous temperature measurement value.

The use of cold saline for perfusion cooling in the radio frequency ablation system 100 can cause the temperature around the ablation electrode 120 to drop rapidly. To maintain a normal operating temperature at the ablation electrode 120, the radio frequency power source 110 can be controlled to continue the power supply to the ablation electrode 120 when the temperature measurement value at the current moment is lower than or equal to the temperature measurement value at the previous moment. This can prevent the temperature of the ablation electrode 120 from falling below the permissible temperature and keep it within a constant range. By avoiding destabilization in temperature control and excessive heating of the pulmonary artery target, the system can also prevent the ablation electrode 120 from becoming too cold, which may negatively impact the therapeutic effect of the radio frequency ablation. Consequently, temperature fluctuations of the ablation electrode 120 can be further reduced, ensuring a stable control within a specific range.

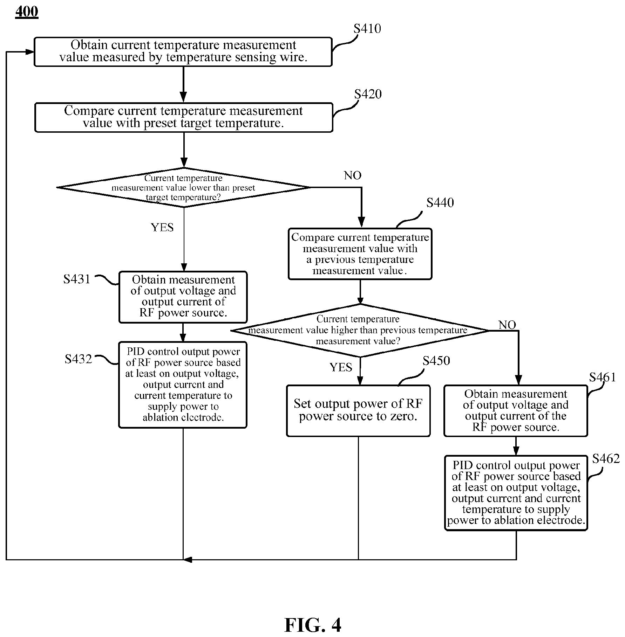

**FIG. 4** illustrates a flowchart of a temperature control method 400 for a pulmonary artery radio frequency ablation system according to embodiments of the present disclosure. As shown in **FIG. 4****,** the method 400 can comprise steps S410 to steps S462. Description on steps that are the same or similar to those in method 200, as described hereinabove with respect to **FIG. 2****,** or method 300, as described hereinabove with respect to **FIG. 3****,** will be omitted for brevity.

In some embodiments, in the method 400, the step of controlling the radio frequency power source 110 to supply a power to the ablation electrode 120 can comprise, in step S431 (or step S461), obtaining a measurement of an output voltage and an output current of the radio frequency power source 110, and in step S432 (or step S462), PID controlling an output power of the radio frequency power source 110 based at least on the output voltage, the output current and the current temperature to supply power to the ablation electrode 120.

The output power of the radio frequency power source 110 can be determined by measuring the output voltage and output current of the radio frequency power source 110. Subsequently, based on the current temperature measurement value, the current output power, and a target temperature or a target power, a PID control can be performed on the output power of the radio frequency power source 110. The benefits of PID control can be leveraged to ensure a more stable power output when supplying power to the ablation electrode 120.

With reference to **FIG. 4****,** in some embodiments, controlling the radio frequency power source 110 to suspend the power supply to the ablation electrode 120 can comprise, in step S450, setting the output power of the radio frequency power source to zero. By setting the output power of the radio frequency power source to zero in a straightforward and user-friendly way, the power supply to the ablation electrode 120 can be precisely suspended. This approach can be beneficial for minimizing temperature fluctuations and maintaining the temperature of the ablation electrode 120 within a specific range.

In some embodiments, the PID controlling the output power of the radio frequency power source in step S432 or step S462 can comprise performing the PID control with a parameter P in a range of 0.1 to 0.2, a parameter I in a range of 0.15 to 0.2, and a parameter D in a range of -0.1 to -0.5 when the current temperature measurement value is lower than or equal to 45 °C.

Through extensive simulations and experimental verifications, the inventors discovered that, given the properties of human pulmonary artery tissue, conventional temperature control approaches tend to destabilize the system when the target temperature of the pulmonary artery is around 45°C. However, when the current temperature measurement value is less than or equal to 45°C, setting the parameter P between 0.1 to 0.2, the parameter I between 0.15 to 0.2, and the parameter D between -0.1 to -0.5 can result in a more stable system response.

In some embodiments, the PID controlling the output power of the radio frequency power source in step S432 or step S462 can comprise performing the PID control with a parameter P in a range of 0.3 to 0.5, a parameter I in a range of 0.1 to 0.18, and a parameter D in a range of -1 to -1.5 when the current temperature measurement value is higher than 45°C.

Similarly, when the current temperature measurement value is higher than 45°C, setting the parameter P between 0.3 to 0.5, the parameter I between 0.1 to 0.18, and the parameter D between -1 to -1.5 can result in a more stable system response.

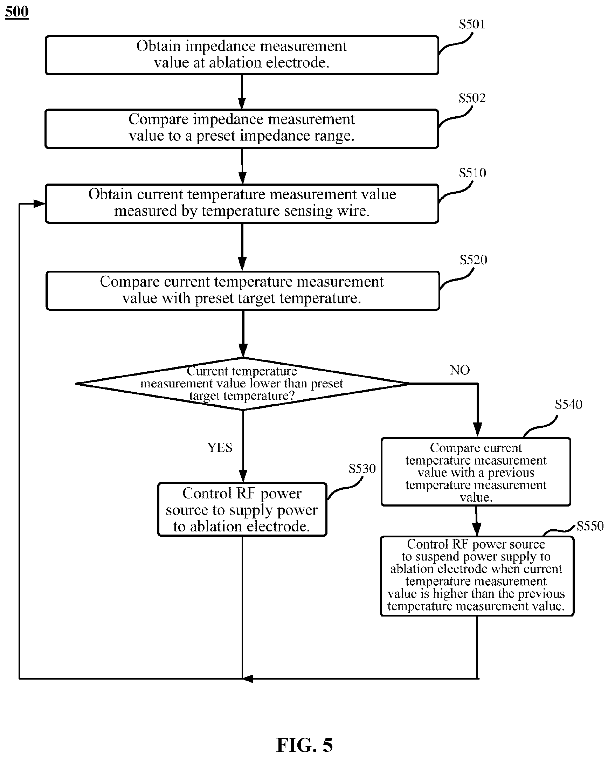

**FIG. 5** illustrates a flowchart of a temperature control method 500 for a pulmonary artery radio frequency ablation system according to embodiments of the present disclosure. As shown in **FIG. 5****,** the method 500 can comprise step S501, step S502 and steps 510 to S50. Description on steps S520 to S550, that are similar to steps S220 to S250 in method 200 as described hereinabove with respect to **FIG. 2****,** will be omitted for brevity.

In some embodiments, method 500 can further comprise, in step S501, obtaining an impedance measurement value at the ablation electrode 120 prior to a first ablation cycle, and in step S502, comparing the impedance measurement value to a preset impedance range.

In some embodiments, in step S 510, the current temperature measurement value measured by the temperature sensing wire 130 can be obtained when the impedance measurement value falling within the preset impedance range.

As a result, prior to starting the first ablation cycle, it is possible to verify whether it is permissible to initiate the radio frequency ablation procedure by determining whether the impedance measurement value at the ablation electrode 120 aligns with the preset impedance range criteria. This can prevent a condensation of the target tissue on the surface of the ablation electrode 120 after denaturation or carbonization of the target tissue site, which may adversely affect the output of radio frequency energy. For example, the preset impedance range can be set between 100 and 300 ohms. The subsequent operation can only proceed when the impedance measurement at the ablation electrode 120 falls within the range. If the impedance measurement value at the electrode 120 is outside the 100 to 300 ohms range, it may indicated denatured or carbonized pulmonary artery tissue, and therefore the subsequent operation should not be performed. This can ensure the safety of the radio frequency ablation procedure.

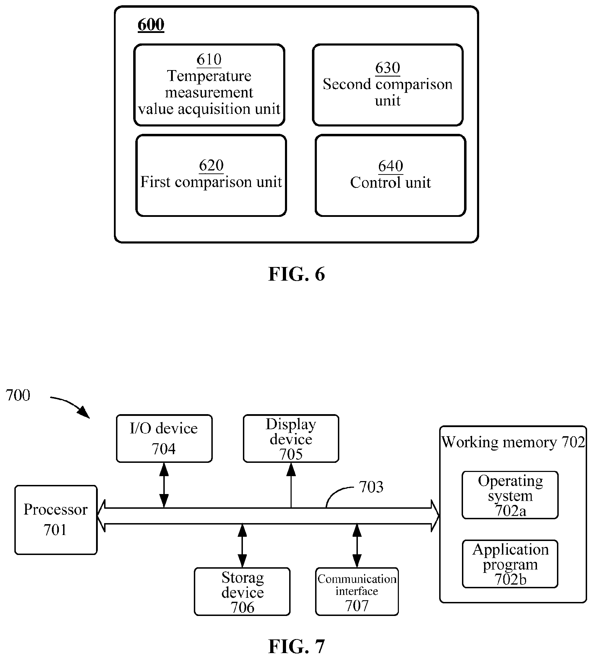

Another aspect of the present disclosure provides a temperature control device for a pulmonary artery radio frequency ablation system. **FIG. 6** illustrates a block diagram of a temperature control device 600 for a pulmonary artery radio frequency ablation system according to embodiments of the present disclosure.

As shown in **FIG. 6****,** the temperature control device 600 can comprise a temperature measurement value acquisition unit 610, a first comparison unit 620, a second comparison unit 630 and a control unit 640.

In some embodiment, the temperature measurement value acquisition unit 610 can be configured to obtain a current temperature measurement value measured by the temperature sensing wire 130 during each of a plurality of ablation cycles. The first comparison unit 620 can be configured to compare the current temperature measurement value with a preset target temperature during each of the plurality of ablation cycles. The control unit 640 can be configured to control the radio frequency power source 110 to supply a power to the ablation electrode 120 during the ablation cycle when the current temperature measurement value is lower than the preset target temperature. The second comparison unit 630 can be configured to compare the current temperature measurement value with a previous temperature measurement value during the ablation cycle when the current temperature measurement value is higher than or equal to the preset target temperature. The control unit 640 can be further configured to control the radio frequency power source 110 to suspend a power supply to the ablation electrode 120 when the current temperature measurement value is greater than the previous temperature measurement value.

In some embodiments, the control unit 640 can be further configured to control the radio frequency power source 110 to supply a power to the ablation electrode 120 when the current temperature measurement value is lower than or equal to the previous temperature measurement value.

In some embodiments, the control unit 640 can be further configured to obtain a measurement of an output voltage and an output current of the radio frequency power source 110, and PID control an output power of the radio frequency power source 110 based at least on the output voltage, the output current and the current temperature to supply power to the ablation electrode 120.

In some embodiments, the control unit 640 may be further configured to set the output power of the radio frequency power source 110 to zero.

n some embodiments, the control unit 640 can be further configured to perform the PID control with a parameter P in a range of 0.1 to 0.2, a parameter I in a range of 0.15 to 0.2, and a parameter D in a range of -0.1 to -0.5 when the current temperature measurement value is lower than or equal to 45°C.

In some embodiments, the control unit 640 can be further configured to perform the PID control with a parameter P in a range of 0.3 to 0.5, a parameter I in a range of 0.1 to 0.18, and a parameter D in a range of -1 to -1.5 when the current temperature measurement value is higher than 45°C.

In some embodiments, the temperature control device 600 can further comprise an impedance detection unit (not shown in the figures). The impedance detection unit can be configured to obtain an impedance measurement value at the ablation electrode prior to a first ablation cycle and compare the impedance measurement value to a preset impedance range. In some embodiments, the temperature measurement value acquisition unit 610 can be further configured to obtain the current temperature measurement value measured by the temperature sensing wire 130 when the impedance measurement value falling within the preset impedance range.

Another aspect of the present disclosure provides an electronic device. In some embodiments, the electronic device can comprise at least one processor and a memory in communication with the at least one processor. The memory can store therein instructions executable by the at least one processor that, upon execution by the at least one processor, implements the temperature control method for pulmonary artery radio frequency ablation system.

Another aspect of the present disclosure provides a non-transitory computer-readable storage medium storing computer instructions, wherein the computer instructions are executable to cause a computer to implement the temperature control method for pulmonary artery radio frequency ablation system.

Another aspect of the present disclosure provides a computer program product comprising a computer program, upon execution by the at least one processor, implements the temperature control method for pulmonary artery radio frequency ablation system.

**FIG. 7** shows a block diagram of an exemplary electronic device 700, which can serve as a hardware example for implementing the embodiments described in this disclosure. These electronic devices can include various types of computers, such as laptops, desktops, workstations, personal digital assistants, servers, blade servers, mainframes, and other suitable computers. They can also include different mobile devices, including personal digital processors, cell phones, smartphones, wearable devices, and similar computing devices. The components, their connections, relationships, and functionalities described here are intended as examples and are not meant to limit the implementations of this disclosure.

**FIG. 7** illustrates a block diagram of an electronic device according to embodiments of the present disclosure. As shown in **FIG. 7****,** the electronic device 700 can comprise at least one processor 701, a working memory 702, an I/O device 704, a display device 705, a storage device 706, and a communication interface 707 that in communication with each other via a system bus 703.

The processor 701 can be a single processing unit or a plurality of processing units, all of which can include a single or plurality of computing units or a plurality of cores. The processor 701 can be implemented as one or more microprocessors, microcomputers, microcontrollers, digital signal processors, central processing units, state machines, logic circuits, and/or any device that manipulates signals based on operational instructions. The processor 701 can be configured to acquire and execute computer-readable instructions stored in the working memory 702, storage device 706, or other computer-readable medium, such as program code for an operating system 702a, program code for an application program 702b, and the like.

The working memory 702 and the storage device 706 are examples of computer-readable storage media for storing instructions that are executed by the processor 701 to carry out the various functions as described hereinabove. The working memory 702 can include both volatile memory and non-volatile memory (e.g., RAM, ROM, etc.). In addition, the storage device 706 can include hard disk drives, solid state drives, removable media, including external and removable drives, memory cards, flash memory, floppy disks, optical disks (e.g., CDs, DVDs), storage arrays, network attached storage, storage area networks, and the like. Both the working memory 702 and the storage device 706 can be collectively referred to herein as a memory or computer-readable storage medium. Both the working memory 702 and the storage device 706 can be a non-transitory medium capable of storing computer-readable, processor-executable program instructions as computer program code, which are executable by the processor 701 as a particular machine configured to perform the operations and functions as described hereinabove.

The I/O device 704 can include an input device and/or an output device. The input device can be any type of device capable of inputting information to the electronic device 700. The input device can include, but is not limited to, a mouse, a keyboard, a touch screen, a track pad, a trackball, a joystick, a microphone, and/or a remote control. The output device can include any type of device capable of presenting information. The output device can include, but is not limited to, including a video/audio output terminal, a vibrator, and/or a printer.

The communication interface 707 can allow the electronic device 700 to exchange information/data with other devices over a computer network, such as the Internet, and/or various telecommunication networks. The communication interface 707 can include, but is not limited to, modems, network cards, infrared communication devices, wireless communication transceivers and/or chipsets, such as Bluetooth^{™} devices, 802.11 devices, WiFi devices, WiMax devices, cellular communication devices and/or the like.

The application program 702b in the working memory 702 can be loaded to execute the various methods and processes described hereinabove. In some embodiments, some or all of the computer program can be loaded and/or installed onto the electronic device 700 via the storage device 706 and/or the communication interface 707. When the computer program is loaded and executed by the processor 701, one or more steps of the temperature control method described hereinabove for a pulmonary artery radio frequency ablation system can be performed.

Various embodiments of the systems and techniques described hereinabove can be implemented in digital electronic circuit systems, integrated circuit systems, field programmable gate arrays (FPGAs), application-specific integrated circuits (ASICs), application-specific standard products (ASSPs), systems of systems-on-a-chip (SOCs), load-programmable logic devices (CPLDs), computer hardware, firmware, software, and/or combinations thereof implementations. The embodiment can include an implementation in one or more computer programs that is executable and/or interpretable on a programmable system comprising at least one programmable processor, which can be a dedicated or general-purpose programmable processor, that can receive data and instructions from the storage system, the at least one input device, and the at least one output device, and transmit the data and instructions to the storage system, the at least one input device, and the at least one output device.

Program code for implementing the methods of the present disclosure can be written in any combination of one or more programming languages. Such program code can be provided to a processor or controller of a general-purpose computer, special-purpose computer, or other programmable data processing device such that the program code, when executed by the processor or controller, causes the functions/operations as described with reference to the flowchart and/or block diagram to be implemented. The program code can be executed entirely on the machine, partially on the machine, partially on the machine as a stand-alone software package and partially on a remote machine, or entirely on a remote machine or server.

In the context of the present disclosure, a machine-readable medium can refer to a tangible medium that can contain or store a program for use by or in combination with an instruction execution system, device, or apparatus. The machine-readable medium can be a machine-readable signal medium or a machine-readable storage medium. The machine-readable medium can include, but is not limited to, an electronic, magnetic, optical, electromagnetic, infrared, or semiconductor system, device, or apparatus, or any suitable combination of the foregoing. Additional specific examples of machine-readable storage media can include electrical connections based on one or more wires, portable computer disks, hard disks, random access memory (RAM), read-only memory (ROM), erasable programmable read-only memory (EPROM or flash memory), optical fibers, convenient compact disk read-only memory (CD-ROM), optical storage devices, magnetic storage devices, or any combination thereof.

To provide interaction with a user, the systems and techniques described hereinabove can be implemented on a computer having a display device (e.g., a CRT (cathode ray tube) or LCD (liquid crystal display) monitor) for displaying information to the user and a keyboard and a pointing device (e.g., a mouse or a trackball) by which the user can provide input to the computer. Other types of devices can also be used to provide interaction with the user. For example, the feedback provided to the user can be any form of sensory feedback (e.g., visual feedback, auditory feedback, or tactile feedback). The input from the user can be received in any form, including acoustic, voice, or tactile input.

The systems and techniques described hereinabove can be implemented in a computing system having a back-end component (e.g., as a data server), or a computing system having a middleware component (e.g., an application server), or a computing system having a front-end component (e.g., a user's computer that has a graphical user interface or a web browser through which a user can interact with the systems and techniques described herein), or a computing system having any combination of the back-end, middleware, or front-end components. The components of the system can be interconnected by digital data communications (e.g., a communications network) in any form or medium. Examples of communication networks can include local area networks (LANs), wide area networks (WANs), and the Internet.

A computing system can include a client and a server. A client and a server are generally remote from each other and interact over a communication network. The client-server relationship can be created by computer programs that are running on corresponding computers and have a client-server relationship with each other.

It should be understood that the steps can be reordered, added, or removed in various ways. For example, the steps as described in the present disclosure can be performed in parallel, or sequentially or in a different order, as long as the results desired by the technical solutions achieved.

Although the embodiments or examples of the present disclosure are described with reference to the drawings, the methods, systems, and devices as described hereinabove are merely illustrative. The scope of the present disclosure is defined by the granted claims and their equivalents, not by the specific embodiments or examples. Various elements in the embodiments or examples can be omitted or can be replaced by their equivalents. Additionally, the steps can be performed in an order different than described. Furthermore, elements from different embodiments or examples can be combined in various ways. As technology evolves, the described elements can be replaced by their future equivalents.

## Claims

1. A temperature control method for a pulmonary artery radio frequency ablation system, the radio frequency ablation system comprising a radio frequency power source, an ablation electrode and a temperature sensing wire coupled with the ablation electrode, the method comprising:
during each of a plurality of ablation cycles:
obtaining a current temperature measurement value measured by the temperature sensing wire; and
comparing the current temperature measurement value with a preset target temperature;
controlling the radio frequency power source to supply a power to the ablation electrode when the current temperature measurement value is lower than the preset target temperature during the ablation cycle; and
comparing the current temperature measurement value with a previous temperature measurement value during the ablation cycle when the current temperature measurement value is higher than or equal to the preset target temperature, and controlling the radio frequency power source to suspend a power supply to the ablation electrode when the current temperature measurement value is higher than the previous temperature measurement value.

2. The method of claim 1, further comprising controlling the radio frequency power source to supply a power to the ablation electrode when the current temperature measurement value is lower than or equal to the previous temperature measurement value.

3. The method of claim 1 or 2, wherein controlling the radio frequency power source to supply a power to the ablation electrode comprises:
obtaining a measurement of an output voltage and an output current of the radio frequency power source; and
PID controlling an output power of the radio frequency power source based at least on the output voltage, the output current and the current temperature to supply power to the ablation electrode.

4. The method of claim 3, wherein controlling the radio frequency power source to suspend the power supply to the ablation electrode comprises setting the output power of the radio frequency power source to zero.

5. The method of claim 3, wherein the PID controlling the output power of the radio frequency power source comprises performing the PID control with a parameter P in a range of 0.1 to 0.2, a parameter I in a range of 0.15 to 0.2, and a parameter D in a range of -0.1 to -0.5 when the current temperature measurement value is lower than or equal to 45°C.

6. The method of claim 3, wherein the PID controlling the output power of the radio frequency power source comprises performing the PID control with a parameter P in a range of 0.3 to 0.5, a parameter I in a range of 0.1 to 0.18, and a parameter D in a range of -1 to -1.5 when the current temperature measurement value is higher than 45°C.

7. The method of claim 1 or 2, further comprising:
obtaining an impedance measurement value at the ablation electrode prior to a first ablation cycle; and
comparing the impedance measurement value to a preset impedance range, and
wherein obtaining the current temperature measurement value measured by the temperature sensing wire comprises:
obtaining the current temperature measurement value measured by the temperature sensing wire when the impedance measurement value falling within the preset impedance range.

8. A temperature control device for a pulmonary artery radio frequency ablation system, the radio frequency ablation system comprising a radio frequency power source, an ablation electrode and a temperature sensing wire coupled with the ablation electrode, the device comprising a temperature measurement value acquisition unit, a first comparison unit, a second comparison unit and a control unit,
wherein the temperature measurement value acquisition unit is configured to obtain a current temperature measurement value measured by the temperature sensing wire during each of a plurality of ablation cycles;
wherein the first comparison unit is configured to compare the current temperature measurement value with a preset target temperature during each of the plurality of ablation cycles;
wherein the control unit is configured to control the radio frequency power source to supply a power to the ablation electrode during the ablation cycle when the current temperature measurement value is lower than the preset target temperature;
wherein the second comparison unit is configured to compare the current temperature measurement value with a previous temperature measurement value during the ablation cycle when the current temperature measurement value is higher than or equal to the preset target temperature; and
wherein the control unit is further configured to control the radio frequency power source to suspend a power supply to the ablation electrode when the current temperature measurement value is greater than the previous temperature measurement value.

9. The device of claim 8, wherein the control unit is further configured to control the radio frequency power source to supply a power to the ablation electrode when the current temperature measurement value is lower than or equal to the previous temperature measurement value.

10. The device of claim 8 or 9, wherein the control unit is further configured to:
obtain a measurement of an output voltage and an output current of the radio frequency power source; and
PID control an output power of the radio frequency power source based at least on the output voltage, the output current and the current temperature to supply power to the ablation electrode.

11. The device of claim 10, wherein the control unit is further configured to set the output power of the radio frequency power source to zero.

12. The device of claim 10, wherein the control unit is further configured to perform the PID control with a parameter P in a range of 0.1 to 0.2, a parameter I in a range of 0.15 to 0.2, and a parameter D in a range of -0.1 to -0.5 when the current temperature measurement value is lower than or equal to 45°C.

13. The device of claim 10, wherein the control unit is further configured to perform the PID control with a parameter P in a range of 0.3 to 0.5, a parameter I in a range of 0.1 to 0.18, and a parameter D in a range of -1 to -1.5 when the current temperature measurement value is higher than 45°C.

14. The device of claim 8 or 9, further comprising an impedance detection unit configured to:
obtain an impedance measurement value at the ablation electrode prior to a first ablation cycle; and
compare the impedance measurement value to a preset impedance range,
wherein the temperature measurement value acquisition unit is further configured to obtain the current temperature measurement value measured by the temperature sensing wire when the impedance measurement value falling within the preset impedance range.

15. An electronic device comprising:
at least one processor; and
a memory in communication with the at least one processor;
wherein the memory stores therein instructions executable by the at least one processor that, upon execution by the at least one processor, implements the method of any one of claims 1-7.

16. A non-transitory computer-readable storage medium storing computer instructions, wherein the computer instructions are executable to cause a computer to implement the method of any one of claims 1-7.

17. A computer program product comprising a computer program, upon execution by the at least one processor, implements the method of any one of claims 1-7.
